(19) Europäisches Patentamt
European Patent Office
Office européen des brevets

(11) Publication number: **0 161 459**
**B1**

# (12) EUROPEAN PATENT SPECIFICATION

(45) Date of publication of patent specification: **25.05.88**

(51) Int. Cl.$^4$: **C 07 C 93/04,** C 07 C 43/11, C 07 C 41/03, C 11 D 1/825

(21) Application number: **85104039.4**

(22) Date of filing: **03.04.85**

## (54) Alcohol-amine alkoxylates.

(30) Priority: **04.05.84 US 607036**

(43) Date of publication of application:
**21.11.85 Bulletin 85/47**

(45) Publication of the grant of the patent:
**25.05.88 Bulletin 88/21**

(84) Designated Contracting States:
**BE DE FR GB SE**

(58) References cited:
**US-A-3 446 848**
**US-A-3 696 057**

(73) Proprietor: **Conoco Inc.**
**1000 South Pine Street P.O. Box 1267**
**Ponca City Oklahoma 74603 (US)**

(72) Inventor: **Yang, Kang**
**2501 Robin Road**
**Ponca City Oklahoma 74601 (US)**

(74) Representative: **Patentanwälte Dipl.-Ing. A. Grünecker, Dr.-Ing. H. Kinkeldey, Dr.-Ing. W. Stockmair,**
**Dr. rer. nat. K. Schumann, Dipl.-Ing. P.H. Jakob,**
**Dr. rer. nat. G. Bezold Maximilianstrasse 58**
**D-8000 München 22 (DE)**

**EP 0 161 459 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to amine-alcohol surfactants and a method for producing same as defined in the claims.

It is known in the art that aqueous solutions of cleaning compositions desirably have strong cleaning action. In the theory of detergency, it is believed that in order for an aqueous cleaning system to be effective against oily soil or contaminants, that a hydrophilic and a hydrophobic portion of the surfactant molecule be present in order to effectively maintain the oily soil in the aqueous solution during cleaning. Among such well known cleaners are non-ionic alcohol surfactants, usually alkoxylated, non-ionic surfactants together with an adjunct in order to facilitate cleaning.

Ethoxylation of amines is known using basic catalysts. However such materials produce highly colored products containing appreciable amounts of by-products. These materials are generally considered amphoteric surfactants but are not generally favored because of expense and low effectiveness as compared to non-ionic surfactants.

US—A—3,446,848 discloses a process for the manufacture of a polyether-polyol by reacting an alkylene oxide with a mixture of a polyhydric alcohol having a melting point in excess of 100°C and triethanolamine at a temperature ranging from 50°C to 150°C and at a pressure up to 80 psi. Ethylene oxide may be used as the alkylene oxide.

The surface activity of both alcohol ethoxylates and amine ethoxylates are all well-known, as set forth in Non-Ionic Surfactants, M. J. Schick, Marcell Decker, Inc. New York, (1967). Representative but non-exhaustive examples of the patent art showing ethoxylated amines are U.S. Patents 3,741,912; 3,953,382; 3,696,057 and 4,276,205. However, all these references contain hydrophobic materials such as propylene oxides, or deal with amines containing a sufficiently large number of carbon atoms that the amine itself acts as a hydrophobic material.

Thus all these references conform to accepted surfactant theory in that all these surfactants contain hydrophilic ethylene oxide groups and hydrophobic hydrocarbon chains. When a molecule contains either hydrophobic or hydrophilic groups alone, such molecules are not expected to function as surfactants. Ethoxylates of water soluble amines contain only hydrophilic groups and thus conform to this generally accepted theory.

It is the object of the present invention to provide amine-alcohol surfactants whose surface tension is lower than each individual component alone, and a process for producing same.

Said object is achieved by amine-alcohol surfactants produced by the reaction of a mixture of alcohols and amines with ethylene oxide in the presence of an alkali metal basic catalyst at a temperature of from 90°C to 200°C and a pressure of about 0.5 bar to about 10,13 bars (0.5 to 10 atmospheres) wherein

a) the alcohol is at least one alcohol having only one hydroxyl group and being selected from the group consisting of primary, secondary, linear and a branched alkanols, secondary alcohols, and fluorinated alcohols containing 2 to 30 carbon atoms, and

b) the amine is at least one water soluble amine having a general formula selected from the group consisting of

1) 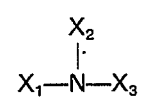

2) 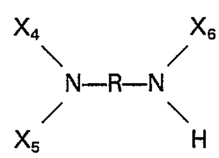

or

3) 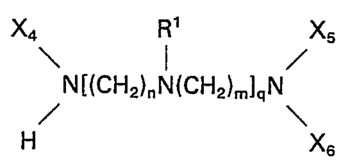

wherein

$X_1$, $X_2$ and $X_3$ are, independently, hydrogen, $CH_2CH_2OH$, $CH_2CH(CH_3)OH$,

$X_4$, $X_5$, $X_6$, R and $R^1$ are, independently, straight, branched, cyclic or aromatic hydrocarbon groups. containing no more than 7 carbon atoms, wherein $X_4$, $X_5$, $X_6$ and $R^1$ can be hydrogen,

n and m are less than or equal to 7, and

q is less than or equal to 20.

These surfactants contain hydrocarbon groups having no more than 7 carbon atoms and being substantially free of hydrophobic groups in the amine portion.

Thus the present invention can be carried out utilizing mixtures of the amines described together with all classes of alcohols described including fluoro alcohols. However, alkanols are preferably used in the present invention. Use of such materials together with water soluble amines containing 7 or less carbon atoms provide a novel class of surfactants having exceptional surfactant properties. As an additional advantage, these materials will have excellent biodegrading unlike the propylene-oxide containing surfactants of the prior art.

Surfactants of the present invention can be prepared by reacting a mixture of alcohols and amines with ethylene oxide in the presence of an alkali metal basic catalyst. Preferred alkali metal catalysts are potassium hydroxide and sodium hydroxide. Mixtures of these catalysts can be used.

In forming the surfactants of the present invention, the ethoxylation is carried out at temperatures ranging from 90°C to 260°C. However, more normal temperatures are from 120°C to 260°C. Commercial operations are normally carried out at a temperature range of from 150°C to 250°C. The most preferred temperatures are from about 150°C to about 190°C.

The alcohols reacted under the process of the present invention contain from 2 to 30 carbon atoms. However, alcohols containing from 4 to 24 carbon atoms are preferred and those containing from 10 to 18 carbon atoms are most preferred.

The process of the present invention can be carried out at ambient pressures. However, pressures up to about 8 bar (100 pounds per square inch gauge) can also be used. While pressure or lack of pressure is not a detriment to the process of the present invention, it is simply more convenient to carry out the reaction in a pressure range of from about atmospheric to about 8 bar.

The ethoxylations of the present invention are carried out using ethylene oxide. Ethylene oxide useful in the present invention need not be specifically purified to rid it of small amounts of other impurities such as propylene oxide and the like, it being understood that excessive amounts of such contaminants could adversely affect the resultant surfactant.

Reaction products can have any desired content of adducted ethylene oxide. For example, ethylene oxide normally comprises from about 30 to about 80% content based on weight of the total surfactant. However, for most purposes, the content of ethylene oxide ranges from about 40% to about 70% by weight. The weight of adducting material present in the reaction is not critical other than the amount necessary to provide sufficient ethylene oxide units to reach the mole adduct level desired for the particular alcohol amine mixture being reacted.

In carrying out the process of the present invention, normally from about 0.05 to about 5.0 weight percent alkali metal basic catalyst are used, based upon the combined weight of alcohol and amine to be reacted. The higher the concentration of catalyst, the more rapidly the reaction goes to completion. However, from a commercial and economical point of view, normally from about 0.1% to about 2% by weight is preferred.

While the instant invention is effective with all classes of alcohols, alkanols are preferred. Of the alkanols, primary, secondary, linear and branched primary alkanols are the most commonly used and are the preferred alcohols of the present invention. Representative examples of such alcohols are those derived by hydrogenation of natural fats and oils, such as CO and TA alcohols, trademark of and sold by Proctor and Gamble Co., such as CO-124N alcohol, CO-1618 alcohol, and TA 1618 alcohol, and ADOL alcohols, trademark of and sold by Ashland Oil Co., such as ADOL 54 alcohol, ADOL 61 alcohol, ADOL 64-alcohol, ADOL 60 alcohol and ADOL 66 alcohol. Alcohols produced by Ziegler chemistry can also be ethoxylated. Examples of these alcohols are ALFOL alcohols, trademark of and sold by Conoco Inc., such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols 8 hydroformylation) produced from olefins. Examples of such alcohols are NEODOL alcohol, trademark of and sold by Shell Oil Co., such as NEODOL 23 alcohol, NEODOL 25 alcohol, NEODOL 45 alcohol; TERGITOL-L, trademark of Union Carbide Corp., such as TERGITOL-L 125 alcohol; LIAL alcohols, trademark of and sold by Liquichimica Co., such as LIAL 125; and isodecyl and tridecyl alcohols, sold by Exxon Corp., such as isodecyl alcohol and tridecyl alcohol. Guerbet alcohols can also be ethoxylated. Representative examples of these alcohols are STANDAMUL alcohol, trademark of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp. Representative examples of such alcohols are 1-decanol; 1-undecanol; 1-dodecanol; 1-tridecanol; 1-tetradecanol; 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 1-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetradecanol; 3-tetradecanol;

4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecenol-1; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-3-tridecanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; 4-ethyl-3-decanol; tetracosanol; hexacosanol; octacosanol; triacontanol; dotriacontanol; hexatriacontanol; 2-decyltetradecanol 2-dodecylhexadecanol.

Also useful in the practice of the present invention are fluoroalcohols. Representative examples of such alcohols are 1,1-dihydro-heptafluorobutylalcohol, trifluoroethanol, 1,1-dihydro-n-undecafluorohexanol; 1,1-dihydro-n-nonadecafluoro-decanol; 1,1-dihydroperfluorodecanol; 1,1-dihydrononafluoroamyl alcohols; 1,1-dihydroundecafluorohexanol, 1,1-dihydropentadecafluorooctanol, 1,1-dihydrononadecafluorodecanol; 2-(perfluoropropyl)ethanol-1; (perfluoroheptyl)ethanol-1; (perfluorodecyl)ethanol-1; 3-(perfluorobutyl)propanol-1; 3-(perfluorooctyl)propanol-1; 3-(perfluorododecyl)propanol-1; 4-(perfluorooctyl)-butanol-1; 5-(perfluoropropyl)pentanol-1; (perfluorobutyl)pentanol-1; (perfluorooctyl)pentanol-1; (perfluorododecyl)pentanol-1; 6-(perfluorodecyl)hexanol-1; 7-(perfluorooctyl)heptanol-1; 8-(perfluorobutyl)-octanol-1; 8-(perfluorooctyl)octanol-1; 8-(perfluorododecyl)octanol-1; 11-(perfluorobutyl)undecanol-1; 11-(perfluorooctyl)undecanol-1; 11-(perfluorododecyl)undecanol-1; 11-(perfluoro-4-ethylcyclohexyl)-undecanol-1; 12-(perfluorobutyl)-dodecanol-1; 12-(perfluorooctyl)dodecanol-1; 12-(perfluorododecyl)-dodecanol-1; N-propyl,N-ethanol(perfluorooctanesulfonamide); N-ethyl,N-hexanol(perfluorooctanesulfonamide); 1,1-dihydro (perfluorobutyl)alcohol; N-ethyl, N-undecanol(perfluorooctanesulfonamide); N-propyl,N-ethanol(perfluorooctanesulfonamide); N-butyl,N-ethanol(perfluorooctanesulfonamide); 3-(perfluorobutyl)propen-2-ol-1; 3-(perfluorooctyl)propen-2-ol-1; 3-(perfluorododecyl)propen-2-ol-1; 4-(perfluorooctyl)buten-3-ol-1; 5-(perfluoropropyl)penten-4-ol-1; 5-(perfluorobutyl)penten-4-ol-1; 5-(perfluorooctyl)penten-4-ol-1; 5-(perfluorododecyl)penten-4-ol-1; 6-(perfluorodecyl)hexen-5-ol-1; 7-(perfluorooctyl)-hepten-6-ol-1; 8-(perfluorobutyl)octen-7-ol-1; 8-(perfluorooctyl)octen-7-ol-1; 8-(perfluorododecyl)octen-7-ol-1; 11-(perfluorobutyl)undecen-10-ol-1; 11-(perfluorooctyl)undecen-10-ol-1; 11-(perfluorododecyl)-undecen-10-ol-1; 11-(perfluoro-4-ethyl cyclohexyl)undecen-10-ol-1; 12-(perfluorobutyl)dodecen-11-ol-1; 12-(perfluorooctyl)dodecen-11-ol-1; 12-(perfluorododecyl)dodecen-11-ol-1.

Thus in the present invention, mixtures of alcohols and water soluble amines are reacted in the presence of an alkali metal catalyst with ethylene oxide to provide a highly unusual surfactant containing essentially no hydrophobic group in the amine portion.

Amines useful in the present invention are those of the general formula

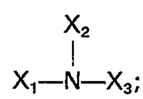

as represented by triethanol amine, diethanol amine, ethanol amine, methyl amine, ethyl amine, pentyl amine, dimethyl amine, methyl pentyl amine, and methyl hexyl amine;

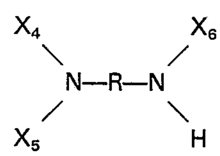

as represented by ethylene diamine, hexamethylene diamine, and 1,2-diamino cyclohexane; and/or

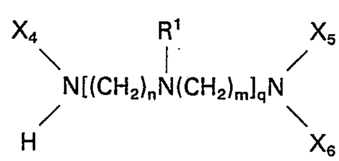

as represented by bis-hexamethylene triamine, and spermine, wherein $X_1$, $X_2$, $X_3$, $X_4$, $X_5$, $X_6$, $R^1$, R, n, m and q are as previously described.

The following examples illustrate the invention.

Example 1

The following reactants and catalyst were added to a 600 ml stainless steel reactor: 10.0 g of a 12 to 14

carbon atom alcohol (Alfol 1214, trademark of and sold by Conoco Inc) 30.0 g triethanol amine and 2.0 g 45% aqueous KOH.

After purging with $N_2$ at 200 ml/min., at 150°C, and for 20 min, 151 g of ethylene oxide (60%) was introduced in 42 min (reaction temperature, 150±5°C, ethylene oxide pressure, 4.1 bar (60 psig). The product was then cooled to 80°C and was neutralized with acetic acid. The product gave surface tension lowering at 25°C as indicated in Figure 1.

Comparison Example 1

An experiment was repeated as in Example 1 with 100 g of 12 to 14 carbon atoms alcohol and 2 g of aqueous KOH. The product, which contained 60% ethylene oxide, gave surface tension reduction as depicted in Figure 1. Figure 1 clearly shows that the ethoxylate of alcohol-amine mixture is more surface active than the ethoxylate of alcohol alone.

Example 2

The following reactants and catalyst were added to a 600 ml stainless steel reactor: 70.0 g of alcohol as described in Example 1, 30.0 g of methylpentamethylenediamine and 2.0 g of 45% aqueous KOH. Ethylene oxide (151 g, 60%) was introduced as described in Example 1. Figure 2 compares surface tension reduction obtained with this product with the reduction obtained with the alcohol ethoxylate prepared in Example 2. Again, the ethoxylate of the alcohol-amine mixture shows higher surface activity than the ethoxylate of alcohol alone.

**Claims**

1. A method for producing amine-alcohol surfactants, containing hydrocarbon groups having no more than 7 carbon atoms and being substantially free of hydrophobic groups in the amine portion, characterized by ethoxylating a mixture of amines and alcohols with ethylene oxide in the presence of an alkali metal basic catalyst, at temperatures from 90°C to 260°C and at pressure up to about 8 bar (100 psi gauge), thereby using

a) as alcohol at least one alcohol having only one hydroxyl group and being selected from the group consisting of primary, secondary, linear and branched alkanols, secondary alcohols, and fluorinated alcohols containing 2 to 30 carbon atoms, and

b) as amine at least one water soluble amine having a general formula selected from the group consisting of

1) 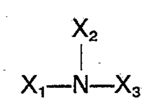

2) 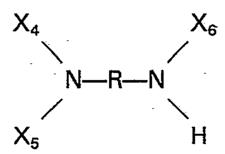

or

3) 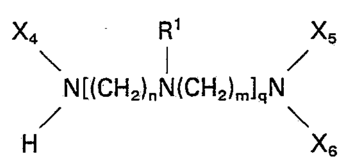

wherein

$X_1$, $X_2$ and $X_3$ are, independently, hydrogen, $CH_2CH_2OH$, $CH_2CH(CH_3)OH$,

$X_4$, $X_5$, $X_6$, R and $R^1$ are, independently, straight, branched, cyclic or aromatic hydrocarbon groups containing no more than 7 carbon atoms, wherein $X_4$, $X_5$, $X_6$ and $R^1$ can be hydrogen,

n and m are less than or equal to 7, and

q is less than or equal to 20.

2. The method as described in claim 1 characterized in that the water soluble amine is at least one material selected from the group consisting of triethanol amine, diethanol amine, ethanol amine, ethylene diamine, hexamethylene diamine, 1,2-diaminocyclohexane, bis-hexamethylene triamine, and spermine.

3. Amine-alcohol surfactants produced by the reaction of a mixture of alcohols and amines with ethylene oxide in the presence of an alkali metal basic catalyst at a temperature of from 90°C to 200°C and a pressure of about 0.5 bar to about 10,13 bars (0.5 to 10 atmospheres) wherein

a) the alcohol is at least one alcohol having only one hydroxyl group and being selected from the group consisting of primary, secondary, linear and branched alkanols, secondary alcohols, and fluorinated alcohols containing 2 to 30 carbon atoms, and

b) the amine is at least one water soluble amine having a general formula selected from the group consisting of

1) 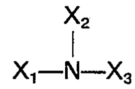

2) 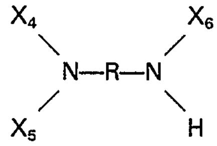

or

3) 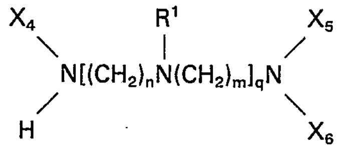

wherein

$X_1$, $X_2$ and $X_3$ are, independently, hydrogen, $CH_2CH_2OH$, $CH_2CH(CH_3)OH$,

$X_4$, $X_5$, $X_6$, R and $R^1$ are, independently, straight, branched, cyclic or aromatic hydrocarbon groups containing no more than 7 carbon atoms, wherein $X_4$, $X_5$, $X_6$ and $R^1$ can be hydrogen,

n and m are less than or equal to 7, and

q is less than or equal to 20.

4. The surfactants according to claim 3, characterized in that they are produced by using as the water soluble amine at least one material selected from the group consisting of triethanol amine, diethanol amine, ethanol amine, ethylene diamine, hexamethylene diamine, 1,2-diamino-cyclohexane, bis-hexamethylene triamine, and spermine.

**Patentansprüche**

1. Verfahren zur Herstellung von Amin-Alkohol-oberflächenaktiven Mitteln, die Kohlenwasserstoffgruppen mit nicht mehr als 7 Kohlenstoffatomen enthalten und im wesentlichen frei von hydrophoben Gruppen in dem Aminteil sind, gekennzeichnet durch Ethoxylieren einer Mischung aus Aminen und Alkoholen mit Ethylenoxid in Gegenwart eines basischen Alkalimetallkatalysators bei Temperaturen von 90°C bis 260°C und einem Druck bis zu etwa 8 bar (100 psi g), worin

a) als Alkohol wenigstens ein Alkohol mit nur einer Hydroxylgruppe, gewählt aus der Gruppe, bestehend aus primären, sekundären, linearen und verzweigten Alkanolen, sekundären Alkoholen und fluorierten Alkoholen mit 2 bis 30 Kohlenstoffatomen, und

b) als Amin wenigstens ein wasserlösliches Amin mit einer allgemeinen Formel, gewählt aus der Gruppe, bestehend aus

1) 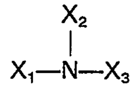

2) 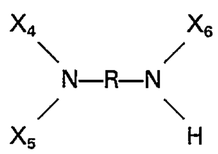

oder

3) 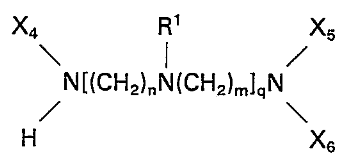

worin

$X_1$, $X_2$ und $X_3$ unabhängig voneinander Wasserstoff, $CH_2CH_2OH$, $CH_2CH(CH_3)OH$ bedeuten und

$X_4$, $X_5$, $X_6$, R und $R^1$ unabhängig voneinander geradkettige, verzweigte, cyclische oder aromatische Kohlenwasserstoffgruppen, die nicht mehr als 7 Kohlenstoffatome enthalten, bedeuten, worin $X_4$, $X_5$, $X_6$ und $R^1$ Wasserstoff sein können,

n und m weniger als oder gleich 7 und

q weniger als oder gleich 20 bedeuten, verwendet wird.

2. Verfahren nach Anspruch 1, dadurch gekennzeichnet, daß das wasserlösliche Amin wenigstens ein Material, gewählt aus der Gruppe, bestehend aus Triethanolamin, Diethanolamin, Ethanolamin, Ethylendiamin, Hexamethylendiamin, 1,2-Diaminocyclohexan, Bis-hexamethylentriamin und Spermin, ist.

3. Amin-Alkohol-oberflächenaktive Mittel, hergestellt durch Reaktion einer Mischung von Alkoholen und Aminen mit Ethylenoxid in Gegenwart eines basischen Alkalimetallkatalysators bei einer Temperatur von 90°C bis 200°C und einem Druck von etwa 0,5 bar bis etwa 10,13 bar (0,5 bis 10 Atmosphären), worin

a) der Alkohol wenigstens ein Alkohol mit nur einer Hydroxylgruppe, gewählt aus der Gruppe, bestehend aus primären, sekundären, linearen und verzweigten Alkanolen, sekundären Alkoholen und fluorierten Alkoholen mit 2 bis 30 Kohlenstoffatomen, ist und

b) das Amin wenigstens ein wasserlösliches Amin mit einer allgemeinen Formel, gewählt aus der Gruppe, bestehend aus

1) 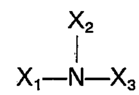

2) 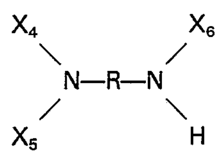

oder

3) 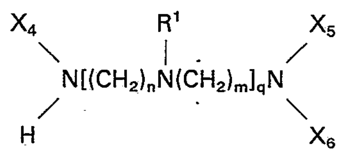

ist, worin

$X_1$, $X_2$ und $X_3$ unabhängig voneinander Wasserstoff, $CH_2CH_2OH$, $CH_2CH(CH_3)OH$ bedeuten,

$X_4$, $X_5$, $X_6$, R und $R^1$ unabhängig voneinander geradkettige, verzweigte, cyclische oder aromatische Kohlenwasserstoffgruppen, welche nicht mehr als 7 Kohlenstoffatome enthalten, bedeuten, worin $X_4$, $X_5$, $X_6$ und $R^1$ Wasserstoff sein können,

n und m weniger als oder gleich 7 und

q weniger als oder gleich 20 bedeuten.

4. Oberflächenaktive Mittel nach Anspruch 3, dadurch gekennzeichnet, daß sie hergestellt werden durch Verwendung von wenigstens einem Material, gewählt aus der Gruppe, bestehend aus Triethanolamin, Diethanolamin, Ethanolamin, Ethylendiamin, Hexamethylendiamin, 1,2-Diaminocyclohexan, Bis-hexamethylentriamin und Spermin, als wasserlösliches Amin.

**Revendications**

1. Procédé de préparation d'agents tensioactifs d'amino-alcool contenant des groupes hydrocarbonés n'ayant pas plus de 7 atomes de carbone et étant pratiquement exempts de groupes hydrophobes dans la partie amine, caractérisé en ce que l'on éthoxyle un mélange d'amines et d'alcools avec de l'oxyde d'éthylène en présence d'un catalyseur basique métallique, à des températures allant de 90°C à 260°C et à une pression pouvant atteindre environ $8 \cdot 10^2$ kPa, dans lequel

a) l'alcool est au moins un alcool ayant seulement un groupe hydroxyle et étant choisi parmi les alcanols linéaires et ramifiés primaires et secondaires, les alcools secondaires et les alcools fluorés contenant de 2 à 30 atomes de carbone, et

b) l'amine est au moins une amine soluble dans l'eau répondant à une formule générale choisie parmi:

1) 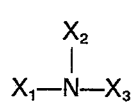

2) 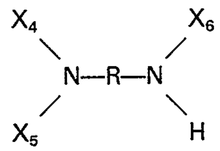

ou

3) 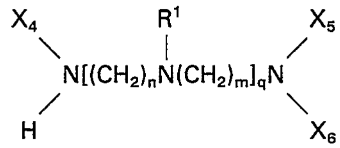

dans lesquelles $X_1$, $X_2$ et $X_3$ sont indépendamment l'hydrogène, $CH_2CH_2OH$, $CH_2CH(CH_3)OH$, et dans lesquelles $X_4$, $X_5$, $X_6$, R et $R^1$ sont indépendamment des groupes hydrocarbonés linéaires, ramifiés, cycliques ou aromatiques ne contenant pas plus de 7 atomes de carbone, $X_4$, $X_5$, $X_6$ et $R^1$ peuvent être l'hydrogène, et dans lesquelles n et m sont inférieurs ou égaux à 7, et q est inférieur ou égal à 20.

2. Procédé suivant la revendication 1, caractérisé en ce que l'amine soluble dans l'eau est au moins une matière choisie parmi la triéthanolamine, la diéthanolamine, l'éthanolamine, l'éthylènediamine, l'hexaméthylène diamine, le 1,2-diamino-cyclohexane, la bis-hexaméthylènetriamine et la spermine.

3. Agents tensio-actifs amine-alcool produits par la réaction d'alcools et d'amines avec l'oxyde d'éthylène en présence d'un mélange d'un métal alcalin comme catalyseur basique, à une température d'environ 90°C à environ 200°C et à une pression d'environ 0,5 $10^2 \cdot$ kPa à environ $10{,}13 \cdot 10^2$ kPa, dans lesquels

a) l'alcool est au moins un alcool choisi parmi les alcanols linéaires et ramifiés primaires et secondaires, les alcools secondaires, et les fluoroalcools ayant de 2 à 30 atomes de carbone,

b) l'amine est au moins une amine soluble dans l'eau dont la formule générale est choisie parmi:

1) 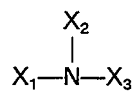

2) 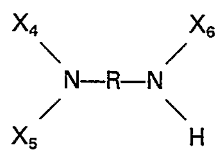

ou

3) 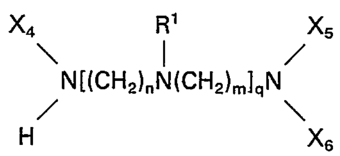

dans lesquelles $X_1$, $X_2$ et $X_3$ sont indépendamment l'hydrogène, $CH_2CH_2OH$, $CH_2CH(CH_3)OH$, et où $X_4$, $X_5$, $X_6$, R et $R^1$ sont indépendamment des groupes hydrocarbonés linéaires, ramifiés, cycliques ou aromatiques ne contenant pas plus de 7 atomes de carbone, $X_4$, $X_5$, $X_6$ et $R^1$ peuvent être l'hydrogène, et dans lesquelles n et m sont inférieurs ou égaux à 7, et q est inférieur ou égal à 20.

4. Agents tensio-actifs selon la revendication 3, caractérisés en ce qu'ils sont obtenus en utilisant comme amine soluble dans l'eau au moins une substance choisie parmi la triéthanolamine, la diéthanolamine, l'éthanolamine, l'éthylènediamine, l'hexaméthylènediamine, le 1,2-diamino-cyclohexane, la bis-hexaméthylènetriamine et la spermine.

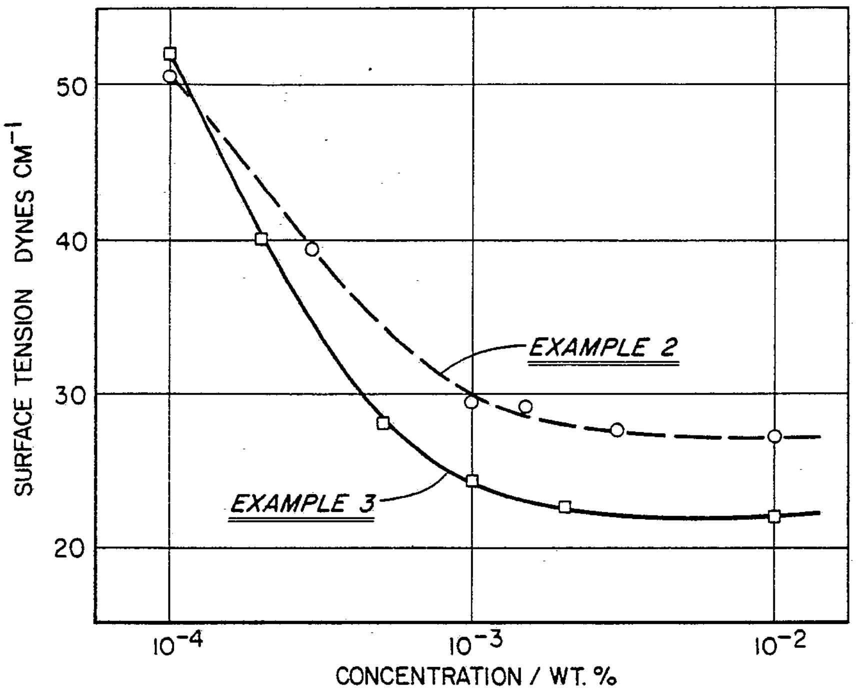
50
40
30
20
SURFACE TENSION DYNES CM$^{-1}$
EXAMPLE 2
EXAMPLE 3
$10^{-4}$
$10^{-3}$
$10^{-2}$
CONCENTRATION / WT. %

FIG. 2

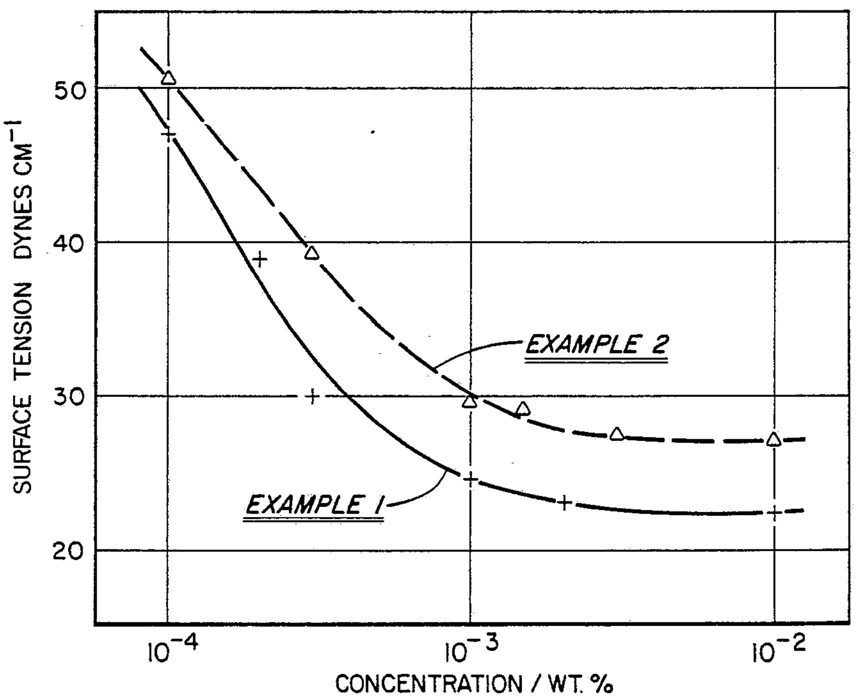
50
40
30
20
SURFACE TENSION DYNES CM$^{-1}$
EXAMPLE 2
EXAMPLE 1
$10^{-4}$
$10^{-3}$
$10^{-2}$
CONCENTRATION / WT. %

FIG. 1